# EUROPEAN PATENT SPECIFICATION

(11) **EP 4 119 030 B1**
(45) Date of publication and mention of the grant of the patent: **28.01.2026**
(21) Application number: 22184202.4
(22) Date of filing: 11.07.2022
(51) Int. Cl.: A61B 1/00, A61B 17/29, A61B 17/34, A61D 1/00

(54) **OPTICAL FORCEPS TO PERFORM LAPAROSCOPIC SURGERIES ON SMALL ANIMALS**
OPTISCHE ZANGE FÜR LAPAROSKOPISCHE EINGRIFFE BEI KLEINE TIERE
PINCE OPTIQUE POUR CHIRURGIES LAPAROSCOPIQUES SUR DE PETITS ANIMAUX

(30) Priority: 15.07.2021 IT 202100018764
(43) Date of publication of application: 18.01.2023
(73) Proprietor: Università degli Studi di Bari "Aldo Moro", 70121 Bari (BA) (IT)
(72) Inventor: LACITIGNOLA, Luca, I-70010 (IT)
(74) Representative: Trupiano, Federica

(56) References cited:
- WO-A1-2019/192068
- AU-A4- 2012 100 339
- DE-U1- 202018 102 182
- US-A- 5 626 608
- US-A1- 2012 157 772
- US-A1- 2016 157 701
- US-A1- 2017 296 319
- US-A1- 2020 155 196
- US-A1- 2021 127 959

## Description

### Summary of the Invention

The present invention relates to a laparoscopic device as defined in claim 1 that allows laparoscopic surgeries to be performed in the veterinary field, and particularly on small animals, allowing to reduce the total number of access points and to avoid the suspension of the organs by sutures. Said device is particularly useful in the case of ovariectomy surgery.

### Background art

Laparoscopy is a minimally invasive surgical technique that has revolutionized many procedures, both diagnostic and surgical, because it allows not only to study organs and tissues inside the abdomen and pelvis but also to perform surgeries which are an alternative to traditional ones, with significant benefits for the patient. Said benefits are mainly dictated by the fact that the surgical procedures performed laparoscopically can be completed by carrying out much smaller incisions, unlike traditional "open" surgery, which requires larger incisions and cuts.

Thus, compared with the traditional surgery, this laparoscopic procedure allows faster recovery to optimal patient condition, less post-surgical pain, smaller scars, lower risk of infection and shorter hospitalization.

In addition to specific surgical instruments, an instrument equipped with optics that is introduced into the operation site through a small cut is also used in this type of laparoscopic surgeries. Said instrument, also called simply "optics", is usually equipped with a light source and a high-definition video camera, which projects the images on a monitor thus allowing the surgeon to observe enlarged details of organs and tissues in the surgical field.

The laparoscopic surgical techniques are also now widely used in the veterinary field, especially in the case of small animals. In particular, one of the most common surgical operations performed with this technique is the ovariectomy (ovary removal) in dogs and cats, a surgical operation that can be performed according to different techniques. In the so-called "three-portal" technique, two incisions are used for the entry of operative instruments (grasping forceps and coagulation devices) and a third incision that allows the entry of the optics (video camera). In this technique, the grasping forceps lifts the organ to be removed while the coagulation instrument is used to dissect the ovarian pedicle and vascular structures. All of this is done thanks to the view provided by the optics inserted in the third portal.

The need to make the surgical surgeries increasingly less invasive has led to the development of surgical techniques that provide for the use of as few portals (and therefore as many instruments) as possible. Therefore, "two-portal" or "single-portal" techniques have been described in recent years. **In** the "two-portal" technique, one fixed inlet is used for the optics (video camera) while, through the other inlet, the different surgical instruments are alternately inserted. **In** this case, the ovary to be removed must be suspended in advance from the abdominal wall by transcutaneous sutures, in order to uncover and visualize the ovarian pedicle as well as the attached vascular structures. Thus, only after this step it will be possible to perform the dissection by means of the appropriate coagulation instruments introduced into the surgical site from the same portal where the instruments for suturing and suspending the organ had previously been introduced. In the "single portal" technique, on the other hand, a special optics equipped with an operational channel is used, which also in this case allows the introduction of only one operational instrument at a time. Therefore, even with this technique there is a suspension step of the ovary on the abdominal wall to allow the visualization and dissection of the pedicle and vessels.

These techniques, while providing reduced invasiveness, are nonetheless not without technical difficulties and/or disadvantages and provide for the use of expensive instrumentation, such as specific optics with operative channel.

It should also be kept in mind that the ovarian suspension step can be particularly complicated in some subjects. The needle used for suturing could damage the vasculature of the organ, leading to bleeding that will need to be stopped and monitored, thus prolonging the surgery time. Furthermore, the needle could break or be damaged during its passage from the skin into the abdominal cavity and/or during its extraction, causing further problems.

The reduction in the number of portals and surgical instruments used simultaneously, which on the one hand makes the surgical operation less invasive, also results in a reduction in the tissue maneuverability. Finally, among the disadvantages it should be taken into account that the two- or single-portal techniques require significantly increased surgical time and have longer learning curves than the classic three-portal technique.

US20160157701 describes an endoscopic instrument that comprises a cylindrical tube in which the lens system can be inserted and, at its distal end, has an instrument made in the form of forceps, which can be operated by means of a handle arranged at the proximal end of the tube, by a transmission element designed as a pulling system. The forceps are in the field of view of an endoscope lens system guided through the tube and allow to monitor the endoscopic manipulations performed with the forceps. Said instrument is currently on the market for the purpose of performing bronchoscopy surgeries in humans and is designed to accommodate special endoscopes having 5.5 mm diameter and 53 cm length, or 2.9 mm diameter and 36 cm length. Endoscopic optical instruments with these characteristics are not commonly used and, because of their size, are not recommended for use in laparoscopic surgeries performed on small animals.

DE202018102182 U1 describes a medical instrument comprising a rod which defines an "optical vision axis" within a channel in which an instrument can be inserted, and which has, at its distal end, a manipulation device and a handle. D2 describes a medical instrument handling device which can operate by means of an actuation device which has the shape of a handle and is arranged in the proximal portion of the shaft.

US2012/157772 discloses an accessory capable of enclosing a probe, preferably an optical probe for endoscopy, capable of defining one or more ducts running parallel next to the probe. Among the various possibilities of use of this duct, described in D3, there is one that provides, for example, for the introduction of a medical instrument such as a forceps, a forceps for biopsies or a tourniquet.

US2021/127959 describes a flexible instrument, equipped with a video camera positioned on its tip, and able to be associated with the probe of an endoscope to allow vision during laparoscopic operations.

US2020/155196 describes a complex system for performing operations, which includes the presence of a probe and a power supply, which flow together into the operating area through a single port.

Thus, there is still the need to provide a device for use in the laparoscopic surgeries, particularly surgeries performed in veterinary settings on small animals, which allows a reduction in the number of access points, avoids the need for anchoring the organs to the abdominal wall, is convenient to use and can be compatible with the normal instrumentation already available in the clinic.

### Objects of the invention

An object of the present invention is to provide a novel device for use in laparoscopic surgeries, and particularly in veterinary surgeries involving small animals, that allows a reduction in the number of the operating access points, avoids the need for anchoring the organs to the abdominal cavity and is compatible with the normal instrumentation present in the clinic.

Another object of the present invention is to use the novel device to perform veterinary ovariectomies on small animals.

These and other objects are achieved by a laparoscopic device as defined in claim 1. Embodiments of the invention are recited in the dependent claims.

### Description of the figures

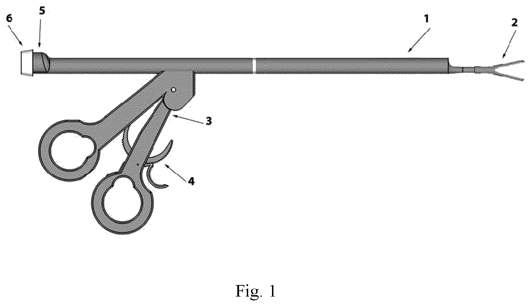

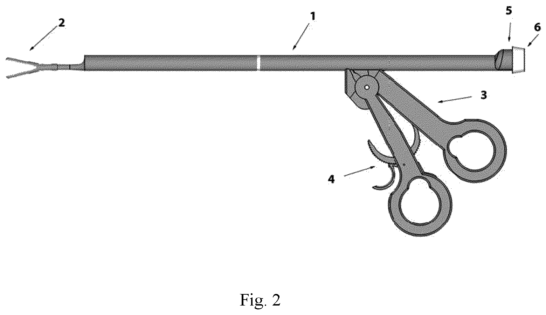

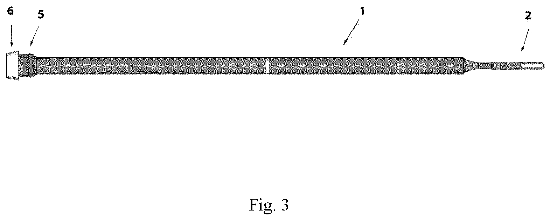

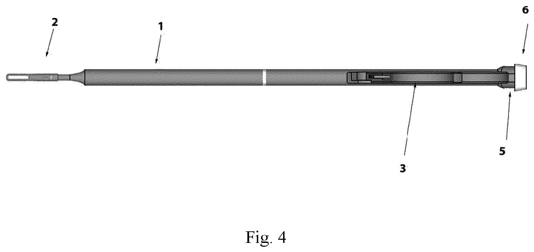

Figures 1, 2, 3, 4 show the surgical instrument of the invention in right side, left side, upper and lower views, respectively. In these representations, the following can be identified: the tool body (1), the grasping forceps (2) with two jaws, the manual activation control (3) of the forceps, equipped with a rack (4) for locking the jaws of the forceps (2) in the desired position, and the optics insertion channel (5) equipped with a gasket (6).

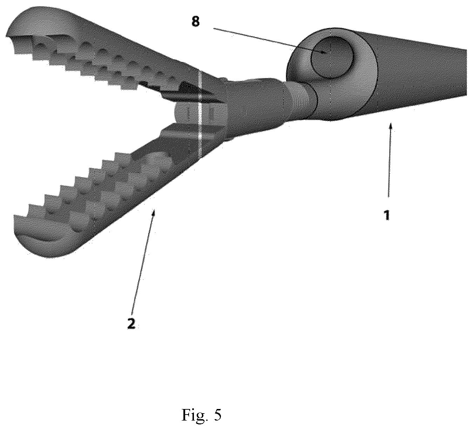

Figure 5 shows the front side of the instrument and in particular, with respect to the device body (1), the geometry of the optics outlet hole (8) and the jaws of the grasping forceps (2).

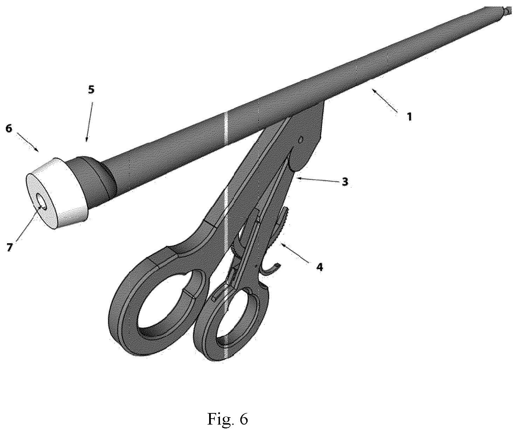

Figure 6 shows the back side of the instrument, through which the optics can be introduced. The body of the device (1), the manual activation control (3) of the forceps equipped with a rack (4) for locking the jaws of the forceps in the desired position, the optics insertion channel (5) equipped with a gasket (6) and the optics inlet hole (7) are highlighted.

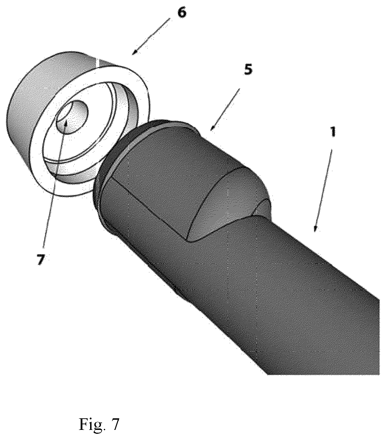

Figure 7 depicts the view, rotated 180° from Figure 6, of the back side of the instrument in which the following are shown: the body (1), the optics insertion channel (5) equipped with a gasket (6) and the optics inlet hole (7).

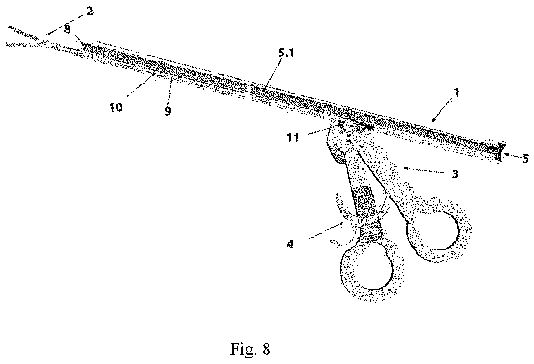

Figure 8 shows a longitudinal section of the instrument. The device body (1), the grasping forceps (2), the manual activation control (3) of the forceps which is equipped with a rack (4) for locking the jaws of the forceps in the desired position, the optics insertion channel (5), the 5.5 mm cylindrical cavity (5.1) running through the main cylindrical body (1), the optics outlet hole (8), the 3 mm cylindrical cavity (9) for sliding the pull rod (10) that activates the jaws of the forceps (2), and the connecting system (11) of the pull rod (10) to the manual activation control (3) of the forceps are depicted.

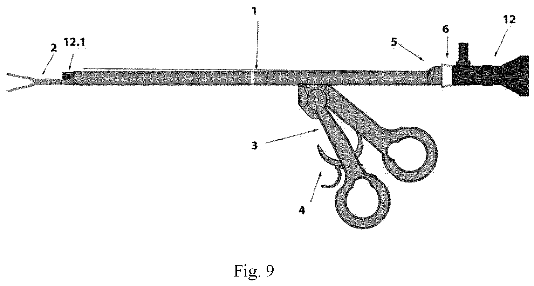

Figure 9 shows the instrument in left side view inside which a rigid optics (12) is inserted, not a subject matter of the present invention. Depicted are: the body of the device (1), the grasping forceps (2), the manual activation control (3) of the forceps equipped with a rack (4) for locking the jaws of the forceps in the desired position, the optics insertion channel (5) equipped with a gasket (6), and the end part of the rigid optics (12.1), not subject matter of the present invention, which protrudes from the front side of the instrument through the channel.

### Description of the invention

Subject matter of the present invention is a novel device for use in the laparoscopic surgical operations, and in particular in the veterinary operations involving small animals, even more particularly in ovariectomies of the dog and/or cat.

In the veterinary practice, and herein, by the expression "small animals" is meant to refer to the category of the pets or companion animals, such as, for example, dogs and cats, as opposed to what are referred to as "livestock animals" such as, for example, cattle, sheep and horses.

The expressions "portals", "incisions", "access points" and "ports" are used alternatively and are meant to refer to the surgical incisions that are made on the animal in order to perform the surgical operation and through which the surgical instruments and optics are introduced into the animal's abdomen.

The device that is subject matter of the present invention, thanks to its dimensional and construction characteristics, allows the number of incisions/portals to be made on the animal, during the surgical operations performed laparoscopically, to be reduced to two. Said reduction of portals is achieved without affecting the maneuverability of the abdominal organs and, more importantly, without the need to suspend said organs with sutures to attach to the abdominal wall itself, which, by their nature, result in increased risks of the surgical operation as a whole as well as increased time to execute it.

In fact, by using the device that is subject matter of the present invention, the veterinarian will not need to suspend an organ from the abdominal wall in order to clear his or her view and the surgical field, but will be able to support the same organ by using said device and simultaneously use the second surgical instrument inserted into the second portal, as per his or her custom, while maintaining his or her view all the time thanks to the optics inserted inside the device of the invention.

Furthermore, the subject matter of the present invention allows to use the endoscopic optical instrumentation commonly found among that included in the normal veterinary laparoscopic surgical equipment, thus avoiding the need to purchase new specific instrumentation (unavoidable in the case of the single-port technique), resulting in significant cost savings as well as time savings thanks to the fact that the surgeon will not be forced to learn how to use a new instrumentation.

In particular, the subject matter of the present invention, which is illustrated in more detail for illustrative but not limiting purpose in the Figures accompanying the present application, is capable of replacing the portal normally dedicated to the optics only during the laparoscopic operations, with an operative channel. Said possibility is determined by the novel device of the invention that comprises both a maneuverable surgical instrument directly involved in performing the operation and the possibility to accommodate, inside it, optics having a 5 mm diameter and length equal to or greater than 29 cm. Said optics is the one most widespread in the field of the veterinary laparoscopic surgery and is considered standard.

According to one of the preferred aspects of the invention, said device has a cylindrical central body (1) with an outer diameter of 11 mm defining a channel having a diameter of 5.5 mm (5) into which the optics (12) can be introduced, said optics not being subject matter of the present invention. Said 5.5 mm diameter channel (5), running the entire length of the main body (1), is unique in type of shape and system of connection to the instrument body itself and allows the insertion of any 5 mm diameter optics, making the device of the invention very versatile.

At the front end of the tool body there are grasping forceps (2) connected to the main body. Said grasping forceps (2), in their closed conformation, preferably have a diameter between 4 and 6 mm, more preferably of 5 mm; a length preferably between 50 and 70 mm, more preferably of 60 mm; and are made of materials known to the skilled in the art, preferably surgical steel or a high temperature resistant plastic material, more preferably 316L surgical steel.

Above the grasping forceps (2), on the same tip as the front end of the device, there is the outlet hole (8) for the optics which, inserted into the cylindrical channel (5) from the hole (7) being on the gasket (6) that closes the rear end of the device, runs through the entire device up to the tip (Figure 5).

The grasping forceps (2) are integrally part of the device of the invention and are constituted by two grips that can be activated by a manual control (3). Said manual control (3) is able to make the two grips move and, consequently, make the grasping forceps (2) open and close, which can remain locked in the desired position thanks to the presence of a rack (4). The movement of the manual control (3) is transmitted to the grasping forceps (2) via a pulling system (10) that runs through the main cylindrical body (1) along a second hollow cylindrical channel (9) having a diameter of 3 mm, located below the channel intended to accommodate the optics.

According to a preferred aspect of the invention, the manual control (3), preferably of a size suitable to accommodate the operator's hand, e.g., 120 mm long, 17 mm wide and 7.8 mm thick, is constituted by a handle into which two fingers of the operator can be inserted which, by moving them away or closer together, transmits the opening and/or closing movement to the grasping forceps located at the opposite end of the device of the invention by means of the pulling system (10), preferably constituted by a 316L surgical steel cable having a diameter of 1.5 mm. The rack (4), which is in the device of the invention, is constituted by a gear which can be activated and deactivated by the operator and allows the grasping forceps (2) to be locked in the desired position and unlocked when necessary. Preferably, said rack (4) is operated by the operator by means of a crescent shaped structure located on the handle of the manual control (3), by which the surgeon, who is holding the device, can engage or disengage the lock on the pulling system (10) and, as a result, can adjust the lock on the position of the grasping forceps (2).

The special configuration of the manual control (3) and rack (4), previously described and exemplified in the figures, particularly in Figure 6, means that the instrument can be operated by the operator with one hand only, thus ensuring that the surgeon can employ the other hand to manoeuvre any second instrument inserted through a second portal.

According to a preferred aspect of the invention, the device as a whole is constructed of a material that is easily washable and sterilizable by the normal methods known to the skilled in the art. In particular, the preferred material according to the invention is 316L surgical steel but other materials known to the skilled in the art and suitable for application in the described field such as, for example, high temperature resistant plastic materials may also be used.

The device that is subject matter of the present invention is also equipped with a 12 mm diameter stopper placed at the rear end and made of plastic and sealing material, for example, a silicone resin and/or a natural or synthetic rubber, preferably a silicone resin. Said stopper constitutes the gasket (6) between the inner channel of the device, in which the optics is inserted, and the optics itself, as it is provided with a central hole (7) having a diameter of 5 mm, through which said optics is passed, and with an outer edge through which it engages the body (1) of the device. The presence of the gasket (6) is made necessary, as the abdominal cavity, during laparoscopic surgeries, is insufflated with CO₂ (pneumoperitoneum) at intra-abdominal pressures between 6 and 12 mmHg to allow distension of the abdominal wall and create an adequate working space for the surgical maneuvers. The gasket (6) as designed prevents the leak of carbon dioxide from the insufflated abdomen during the pneumoperitoneum procedure.

Thanks to the construction characteristics of the device subject matter of the present invention, its application in the laparoscopic surgeries allows reducing, as previously specified, the total number of portals required to complete the endoscopic surgical operation. According to a preferred aspect of the invention, the device of the subject matter is particularly useful during the course of veterinary operations on small animals and, even more preferably, during the ovariectomies on dogs and/or cats, by reducing the number of entries required for the instruments and optics to only two instead of the usual three access points. In fact, the special configuration of the device of the subject matter allows all steps of the surgery, particularly the maneuvers on the ovary, to be performed under endoscopic vision and to allow an adequate dissection by the vessel coagulation instrument introduced through the second portal, thus ensuring an excellent control of the operative triangulation and surgical space.

Another of the main advantages over the known art of what is the subject matter of the present invention relates to the possibility of adapting, inside the novel device, any optical system having a diameter of 5 mm, a size considered standard and with which veterinary clinics are already normally equipped. Thus, as previously mentioned, the device of the invention proves to be versatile and allows to eliminate the costs one would have to incur to purchase a special optical system.

Finally, a significant advantage is brought about by the possibility of decreasing the portals required for the endoscopic surgery without the concomitant need for the physician to be forced to learn new operating techniques. **In** the case of the ovariectomy, for example, the veterinarian will not need to suspend the organ from the abdominal wall to clear the view and the surgical field, but will be able to support the organ itself by using the grasping forceps tip (2) and simultaneously cauterize the pedicle and vessels with the second surgical instrument as per his or her custom, all the while maintaining the view thanks to the optics inserted inside the device of the invention, with consequent advantages also in terms of execution time, which will be significantly reduced compared to a traditional laparoscopic surgical technique.

## Claims

1. A laparoscopic device comprising:
a. a hollow cylindrical main body (1) defining a channel (5) running the entire length of said main body (1);
b. openable and closable grasping forceps (2) connected to a front end of said main body (1); and
c. a manual control (3) connected to said main body (1) and operatively connected to said grasping forceps (2);
wherein said channel (5) has a diameter of 5.5 mm for the insertion of a standard endoscopic optical system, whose lens comes out of a hole (8) placed at the same end of the main body (1) of said grasping forceps (2)
and wherein said manual control (3) is placed in a position distal to said grasping forceps (2) and allows said grasping forceps (2) to be opened and closed by means of a pulling system (10), said pulling system (10) running through the main cylindrical body (1) along a second hollow cylindrical channel (9) having a diameter of 3mm, located below the channel (5) intended to accommodate the optics;
and wherein said device comprises a rack (4) connected to said main body (1) constituted by a gear that allows said grasping forceps (2) to be locked in the desired position by acting on said pulling system (10).

2. The laparoscopic device according to claim 1 **characterized in that** said pulling system (10) comprises a 316L surgical steel cable running along said main cylindrical body (1) throughout said second hollow cylindrical channel (9).

3. The laparoscopic device according to claim 1 **characterized in that** said rack (4) is operated by means of a crescent shaped structure placed on said manual control (3).

4. The laparoscopic device according to claim 1, **characterized in that** it comprises a gasket (6) provided with a hole (7) for the passage of said standard optical system, said gasket being positioned to close the inlet of said channel (5) placed at the distal end with respect to said outlet hole (8) of the lens of the optical system.

5. The laparoscopic device according to any one of the preceding claims **characterized in that** it is constituted by a washable and sterilizable material, preferably 316L surgical steel.

6. The laparoscopic device according to any one of the preceding claims **characterized in that** said main body (1) has an outer diameter of 11 mm.

7. The laparoscopic device according to any one of the preceding claims **characterized in that** said grasping forceps (2), in their closed conformation, has a diameter between 4 and 6 mm and a length between 50 and 70 mm.

8. The laparoscopic device according to claim 1, adapted to perform veterinary endoscopic surgeries on small animals, preferably ovariectomies of dog and/or cat.

## Patentansprüche

1. Laparoskopisches Gerät, aufweisend:
a. einen hohlzylindrischen Hauptkörper (1), der einen Kanal (5) definiert, der über die gesamte Länge des Hauptkörpers (1) verläuft;
b. öffnen- und schließbare Greifzangen (2), die mit einem vorderen Ende des Hauptkörpers (1) verbunden sind; und
c. eine manuelle Steuerung (3), die mit dem Hauptkörper (1) verbunden und betriebsmäßig mit den Greifzangen (2) verbunden ist;
wobei der Kanal (5) einen Durchmesser von 5,5 mm für das Einführen eines Standard-Endoskop-Optiksystems aufweist, dessen Linse aus einem Loch (8) austritt, das an demselben Ende des Hauptkörpers (1) der Greifzangen (2) angeordnet ist,
und wobei die manuelle Steuerung (3) in einer zur Greifzange (2) distalen Position angeordnet ist und das Öffnen und Schließen der Greifzangen (2) mittels eines Zugmechanismus (10) ermöglicht, wobei der Zugmechanismus (10) durch den zylindrischen Hauptkörper (1) entlang eines zweiten hohlzylindrischen Kanals (9) mit einem Durchmesser von 3 mm verläuft, der unterhalb des Kanals (5) angeordnet ist, der zur Aufnahme der Optik vorgesehen ist;
und wobei das Gerät eine Zahnstange (4) umfasst, die mit dem Hauptkörper (1) verbunden ist und durch ein Zahnrad gebildet ist, das es ermöglicht, die Greifzangen (2) in der gewünschten Position durch Einwirkung auf den Zugmechanismus (10) zu verriegeln.

2. Laparoskopisches Gerät nach Anspruch 1, **dadurch gekennzeichnet, dass** der Zugmechanismus (10) ein chirurgisches Stahlkabel aus 316L umfasst, das entlang des zylindrischen Hauptkörpers (1) durch den zweiten hohlzylindrischen Kanal (9) verläuft.

3. Laparoskopisches Gerät nach Anspruch 1, **dadurch gekennzeichnet, dass** die Zahnstange (4) mittels einer halbmondförmigen Struktur betätigt wird, die auf der manuellen Steuerung (3) angeordnet ist.

4. Laparoskopisches Gerät nach Anspruch 1, **dadurch gekennzeichnet, dass** es eine Dichtung (6) umfasst, die mit einem Loch (7) für den Durchtritt des Standard-Optiksystems versehen ist, wobei die Dichtung so positioniert ist, dass sie den Einlass des Kanals (5) verschließt, der am distalen Ende in Bezug auf das Austrittsloch (8) der Linse des Optiksystems angeordnet ist.

5. Laparoskopisches Gerät nach einem der vorhergehenden Ansprüche, **dadurch gekennzeichnet, dass** es aus einem waschbaren und sterilisierbaren Material besteht, vorzugsweise chirurgischem Stahl 316L.

6. Laparoskopisches Gerät nach einem der vorhergehenden Ansprüche, **dadurch gekennzeichnet, dass** der Hauptkörper (1) einen Außendurchmesser von 11 mm aufweist.

7. Laparoskopisches Gerät nach einem der vorhergehenden Ansprüche, **dadurch gekennzeichnet, dass** die Greifzangen (2) in ihrer geschlossenen Konformation einen Durchmesser zwischen 4 und 6 mm und eine Länge zwischen 50 und 70 mm aufweisen.

8. Laparoskopisches Gerät nach Anspruch 1, angepasst zur Durchführung veterinär-endoskopischer Eingriffen an Kleintieren, vorzugsweise Ovariektomien von Hund und/oder Katze.

## Revendications

1. Dispositif laparoscopique comprenant :
a. un corps principal cylindrique creux (1) définissant un canal (5) s'étendant sur toute la longueur dudit corps principal (1) ;
b. une pince de préhension (2) pouvant être ouverte et fermée, reliée à une extrémité avant dudit corps principal (1) ; et
c. une commande manuelle (3) reliée audit corps principal (1) et reliée de manière opérationnelle à ladite pince de préhension (2) ;
dans lequel ledit canal (5) a un diamètre de 5,5 mm pour l'insertion d'un système optique endoscopique standard, dont la lentille sort d'un trou (8) placé à la même extrémité du corps principal (1) de ladite pince de préhension (2)
et dans lequel ladite commande manuelle (3) est placée dans une position distale par rapport à ladite pince de préhension (2) et permet d'ouvrir et de fermer ladite pince de préhension (2) au moyen d'un système de traction (10), ledit système de traction (10) traversant le corps cylindrique principal (1) le long d'un deuxième canal cylindrique creux (9) ayant un diamètre de 3 mm, situé sous le canal (5) destiné à recevoir l'optique ; et dans lequel ledit dispositif comprend une crémaillère (4) reliée audit corps principal (1) constituée d'un engrenage qui permet de verrouiller ladite pince de préhension (2) dans la position souhaitée en agissant sur ledit système de traction (10).

2. Dispositif laparoscopique selon la revendication 1, **caractérisé en ce que** ledit système de traction (10) comprend un câble en acier chirurgical 316L s'étendant le long dudit corps cylindrique principal (1) à travers ledit deuxième canal cylindrique creux (9).

3. Dispositif laparoscopique selon la revendication 1, **caractérisé en ce que** ladite crémaillère (4) est actionnée au moyen d'une structure en forme de croissant placée sur ladite commande manuelle (3).

4. Dispositif laparoscopique selon la revendication 1, **caractérisé en ce qu'**il comprend un joint (6) muni d'un trou (7) pour le passage dudit système optique standard, ledit joint étant positionné de manière à fermer l'entrée dudit canal (5) placé à l'extrémité distale par rapport audit trou de sortie (8) de la lentille du système optique.

5. Dispositif laparoscopique selon l'une quelconque des revendications précédentes, **caractérisé en ce qu'**il est constitué d'un matériau lavable et stérilisable, de préférence de l'acier chirurgical 316L.

6. Dispositif laparoscopique selon l'une quelconque des revendications précédentes, **caractérisé en ce que** ledit corps principal (1) a un diamètre extérieur de 11 mm.

7. Dispositif laparoscopique selon l'une quelconque des revendications précédentes, **caractérisé en ce que** ladite pince de préhension (2), dans sa configuration fermée, a un diamètre compris entre 4 et 6 mm et une longueur comprise entre 50 et 70 mm.

8. Dispositif laparoscopique selon la revendication 1, adapté pour réaliser des chirurgies endoscopiques vétérinaires sur des petits animaux, de préférence des ovariectomies chez le chien et/ou le chat.
